# EUROPEAN PATENT APPLICATION

(11) **EP 4 725 497 A1**
(43) Date of publication of application: **15.04.2026**
(21) Application number: 24819573.7
(22) Date of filing: 05.06.2024
(51) Int. Cl.: A61K 38/45, A61K 38/10, A61P 1/02, A61P 9/10, A61P 25/28, A23L 33/17

(54) **COMPOSITION COMPRISING GV1001 FOR PREVENTING OR TREATING PERIODONTAL DISEASES AND DISORDERS CAUSED BY PERIODONTAL DISEASES**

(30) Priority: 08.06.2023 KR 20230073238; 22.09.2023 KR 20230127253; 13.03.2024 KR 20240035114
(71) Applicant: Gemvax & Kael Co., Ltd., Daejeon 34036 (KR)
(72) Inventor: KIM, Sang Jae, Seoul 06360 (KR)
(74) Representative: Stolmár & Partner Patentanwälte PartG mbB
(86) International application number: PCT/KR2024/007696
(87) International publication number: WO 2024/253422

(57) **Abstract**

The present invention relates to a pharmaceutical composition for use in preventing or treating periodontal disease, or atherosclerosis or Alzheimer's disease caused by periodontal disease, more particularly, a pharmaceutical composition comprising a peptide having the amino acid sequence of SEQ ID NO: 1 to prevent or treat the periodontal disease or the atherosclerosis or Alzheimer's disease caused by periodontal disease, so that it is effective in suppressing osteoclastogenesis, suppressing *Porphyromonas gingivalis* colony formation, and suppressing gingipain expression. Further, the pharmaceutical composition is safe to a living body and involves less side effects including abnormal response.

## Description

### [Technical Field]

The present invention relates to a composition including GV1001 peptide for use in preventing or treating periodontal disease, as well as atherosclerosis and Alzheimer's disease caused by periodontal disease.

### [Background Art]

Periodontal disease is a disease that occurs in the tissues surrounding the teeth, such as the gingiva, periodontal ligament, and alveolar bone, and is classified into gingivitis and periodontitis depending on the severity of the disease. Periodontitis is a chronic oral and systemic inflammatory condition that induces immune inflammatory response leading to irreversible changes in the periodontal tissue (periodontium), ultimately resulting in gradual destruction of the alveolar bone and tooth loss. Periodontitis has been reported to be associated with the onset of various systemic diseases, such as rheumatoid arthritis, psoriasis, systemic sclerosis, Alzheimer's disease (AD), and cardiovascular diseases (CVD), including atherosclerosis.

Periodontal disease has a high prevalence rate. In Korea, the prevalence of periodontal disease in adults aged 19 years or older is 29.8%, and the prevalence increases with age. In particular, the prevalence of periodontal disease increases rapidly in those aged 50 years or older.

According to a 2022 report by TechNavio, the market size for periodontal disease treatment is projected to grow by USD 10.61 billion during the period from 2022 to 2026, with a compound annual growth rate (CAGR) of 9.75% during the forecast period.

Therefore, antibacterial and bacteriostatic activities against anaerobic gram-negative bacteria, which are fundamental pathogens of periodontal disease, the removal of toxic bacterial products, and the regeneration of lost periodontal tissue are key factors in the prevention and treatment of periodontal disease.

Current treatments for periodontal disease include improvement of oral hygiene, nonsurgical treatment, and surgical treatment (scaling, root planing, gingival curettage, and periodontal tissue regeneration using reattachment techniques). However, surgical treatment, which is the most effective method, has the limitation that it is usually performed after the disease has progressed, rather than for prevention, so periodontal disease mostly develops into a chronic condition. As adjunctive therapies, systemic antibiotics and local sustained-release formulations have been used. Nevertheless, these approaches often cause side effects due to unnecessary systemic drug delivery, and cases of antibiotic-resistant periodontal pathogens have recently been reported, highlighting the difficulties in preventing and treating periodontal disease.

Accordingly, there is a growing need for preventive and therapeutic agents for periodontal disease that can overcome the limitations of surgical treatment and the drawbacks of antibiotic use.

Meanwhile, GV1001 peptide (having the amino acid sequence of SEQ ID NO: 1), which consists of 16 amino acids selected from the reverse transcriptase of human telomerase (hTERT), has been reported to exhibit anticancer, anti-inflammatory, and anti-oxidant effects, as well as efficacy in alleviating Alzheimer's disease symptoms. Furthermore, toxicity studies have been completed in numerous clinical trials targeting various diseases, and the safety of GV1001 with respect to adverse effects has been demonstrated.

In light of this, the inventors of the present invention conducted extensive studies to investigate the preventive or therapeutic use of GV1001 against periodontal disease and periodontal disease-induced disorders, such as atherosclerosis and Alzheimer's disease. As a result, the inventors confirmed that GV1001 exerts preventive and/or therapeutic effects on periodontal disease and on atherosclerosis and Alzheimer's disease caused by periodontal disease, and thereby completed the present invention.

### [Summary of Invention]

### [Problems to be Solved by Invention]

An object of the present invention is to provide a pharmaceutical composition for preventing or treating periodontal disease, or atherosclerosis or Alzheimer's disease caused by periodontal disease.

Another object of the present invention is to provide a pharmaceutical composition for preventing or treating *Porphyromonas gingivalis-*induced periodontitis.

Yet another object of the present invention is to provide a kit for preventing or treating periodontal disease, or atherosclerosis or Alzheimer's disease caused by periodontal disease.

Still another object of the present invention is to provide a health functional food for preventing or improving periodontal disease, or atherosclerosis or Alzheimer's disease caused by periodontal disease.

A further object of the present invention is to provide a health functional food for preventing or improving *Porphyromonas gingivalis-*induced periodontitis.

### [Means for Solving Problems]

1. A pharmaceutical composition for use in preventing or treating periodontal disease, or atherosclerosis or Alzheimer's disease caused by periodontal disease, including a peptide having the amino acid sequence of SEQ ID NO: 1.
2. The pharmaceutical composition according to the above 1, wherein the periodontal disease is *Porphyromonas gingivalis-*induced periodontitis.
3. The pharmaceutical composition according to the above 1, wherein the pharmaceutical composition prevents or treats periodontal disease by suppressing osteoclastogenesis.
4. The pharmaceutical composition according to the above 1, wherein the pharmaceutical composition prevents or treats periodontal disease by suppressing the expression of gingipain.
5. A kit for use in preventing or treating periodontal disease or atherosclerosis or Alzheimer's disease caused by periodontal disease, including: the pharmaceutical compositions according to any one of the above 1 to 4; and instructions describing a method for preventing or treating periodontal disease or atherosclerosis or Alzheimer's disease caused by periodontal disease.
6. The kit according to the above 5, wherein the method for preventing and treating includes administering the pharmaceutical composition to a subject who has developed, or is at risk of developing, periodontal disease, or atherosclerosis or Alzheimer's disease caused by periodontal disease.
7. A health functional food for use in preventing or improving periodontal disease, or atherosclerosis or Alzheimer's disease caused by periodontal disease, including a peptide having the amino acid sequence of SEQ ID NO: 1.
8. The food according to the above 7, wherein the periodontal disease is *Porphyromonas gingivalis-*induced periodontitis.
9. The food according to the above 7, wherein the health functional food is a health functional food that prevents or improves periodontal disease by suppressing osteoclastogenesis.
10. The food according to the above 7, wherein the health functional food is a health functional food that prevents or improves periodontal disease by suppressing the expression of gingipain.

### [Advantageous effects]

The pharmaceutical composition of the present invention is effective in preventing or treating periodontal disease by suppressing osteoclast formation (osteoclastogenesis)

The pharmaceutical composition of the present invention is effective in preventing or treating atherosclerosis induced by periodontitis by reducing inflammatory cytokines in the blood, lipid deposition in the arterial wall, and CD47 expression in the arterial root.

The pharmaceutical composition of the present invention is effective in preventing or treating Alzheimer's disease induced by periodontitis by reducing inflammatory cytokines and Alzheimer's disease-related biomarkers in brain tissue.

It is expected that the health functional food of the present invention will be effective in preventing or improving periodontal disease by suppressing osteoclast formation.

It is expected that the health functional food of the present invention will be effective in preventing or improving atherosclerosis induced by periodontitis by reducing inflammatory cytokines in the blood, lipid deposition in the arterial wall, and CD47 expression in the arterial root.

It is expected that the health functional food of the present invention will be effective in preventing or improving Alzheimer's disease induced by periodontitis by reducing inflammatory cytokines and Alzheimer's disease-related biomarkers in brain tissue.

It is expected that the pharmaceutical composition, kit, and health functional food of the present invention will be able to provide economic or medical and social support to patients suffering from periodontal disease and their families.

It is expected that the understanding of the pathophysiology of periodontal disease through the present invention will serve as a driving force for the development of new periodontal disease treatments in the future.

### [Brief Description of Drawings]

FIG. 1 shows the results of comparing cytokine mRNA levels to confirm changes in inflammatory cytokines in the periodontal tissues of the control group and the GV1001 administration group.
FIG. 2 shows the results of measuring cytokine expression level by fluorescent staining to confirm changes in inflammatory cytokines in the periodontal tissues of the control group and the GV1001 administration group.
FIG. 3 shows the results of confirming the number of *Porphyromonas gingivalis* colonies and *Porphyromonas gingivalis* LPS, and confirming changes in aggregates of gingipain (Kgp, RgpB) in the gingival tissues of the control group and the GV1001 administration group.
FIG. 4 shows the results of confirming alveolar bone loss using µCT in the control group and the GV1001 administration group.
FIG. 5 shows the results of determining the number of osteoclasts in the alveolar bone by TRAP staining in the control group and the GV1001 administration group.
FIG. 6 shows the results of measuring the degree of osteoclastogenesis at the *in vitro* level.
FIG. 7 shows the results of measuring the amount of inflammatory cytokines in serum to confirm the level of progression of systemic inflammation due to periodontitis in the control group and the GV1001 administration group.
FIG. 8 shows the results of measuring the level of inflammatory cytokine mRNA in arterial tissue to confirm the possibility of progression to atherosclerosis due to periodontitis in the control group and the GV1001 administration group.
FIG. 9 shows the results of measuring the expression level of inflammatory cytokines in arterial tissue by fluorescent staining to confirm the possibility of progression to atherosclerosis due to periodontitis in the control group and the GV1001 administration group.
FIG. 10 shows the results of confirming the number of *Porphyromonas gingivalis* colonies, and changes in aggregates of *Porphyromonas gingivalis* LPS and gingipain (Kgp, RgpB) in the arterial walls of the control group and the GV1001 administration group.
FIG. 11 shows the results of determining the levels of triglycerides, total cholesterol, high-density lipoprotein, low-density lipoprotein, and very-low-density lipoprotein to confirm changes in cholesterol due to chronic inflammation in the control group and the GV1001 administration group.
FIG. 12 shows the results of determining the level of lipid deposition on the wall of the aorta-iliac bifurcation to verify the induction of atherosclerosis by periodontitis in the control group and the GV1001 administration group.
FIG. 13 shows the results of an experiment to confirm whether HUVECs are transformed into EndMT by TNF-α and *Porphyromonas gingivalis* LPS in the control group and the GV1001 administration group.
FIG. 14 shows the results of an experiment to confirm whether ox-LDL is absorbed by TNF-α and *Porphyromonas gingivalis* LPS in the control group and the GV1001 administration group.
FIG. 15 shows the results of measuring CD47 expression levels in the aortic root in the control group and the GV1001 administration group.
FIG. 16 shows the results of determining the CD47 expression levels using human coronary artery smooth muscle cells (HCASMC) at the *in vitro* level.
FIG. 17 shows the results of determining the levels of inflammatory cytokine mRNA in brain tissue to confirm the possibility of progression to Alzheimer's disease due to periodontitis in the control group and the GV1001 administration group.
FIGS. 18 and 19 show the results of determining the levels of cytokine expression by fluorescent staining to confirm the possibility of progression to Alzheimer's disease due to periodontitis in the control group and the GV1001 administration group.
FIG. 20 shows the results of confirming the number of *Porphyromonas gingivalis* colonies, and changes in aggregates of *Porphyromonas gingivalis* LPS and gingipain (Kgp, RgpB) in the cerebral cortex of the control group and the GV1001 administration group.
FIG. 21 shows the results of confirming the number of *Porphyromonas gingivalis* colonies, and changes in aggregates of *Porphyromonas gingivalis* LPS and gingipain (Kgp, RgpB) in the hippocampus of the control group and the GV1001 administration group.
FIG. 22 shows the results of confirming the accumulation of Aβ₄₂ and p-Tau, which are Alzheimer's disease biomarkers, in the brains of the control group and the GV1001 administration group.
FIG. 23 shows the results of confirming the effect of GV1001 on the expression of gingipain (RgpA, RgpB, Kgp).

### [Mode for Carrying out Invention]

The present invention provides a pharmaceutical composition for use in preventing or treating periodontal disease, or atherosclerosis or Alzheimer's disease caused by periodontal disease, which includes a peptide having the amino acid sequence of SEQ ID NO: 1.

In the present invention, a peptide having the amino acid sequence of SEQ ID NO: 1 includes functional equivalents thereof. The term "functional equivalent" refers to a peptide having at least 70%, 80%, 90%, or 95% sequence identity with the amino acid sequence of SEQ ID NO: 1 as a result of addition, substitution, or deletion of amino acids, and exhibiting substantially the same physiological activity as the peptide having the amino acid sequence of SEQ ID NO: 1. "Substantially the same physiological activity" refers to an activity involved in the prevention, improvement, or treatment of periodontal disease.

In the present invention, "periodontal disease" includes gingivitis and periodontitis.

In one embodiment, the periodontal disease may be gingivitis.

In one embodiment, the periodontal disease may be periodontitis.

In one embodiment, the periodontal disease may be *Porphyromonas gingivalis-*induced periodontitis.

*Porphyromonas gingivalis* is a representative bacterium that causes periodontal disease, and causes tissue invasion, etc. using a protease called gingipain.

In the present invention, "prevention" means any act of suppressing or delaying periodontal disease, or atherosclerosis or Alzheimer's disease caused by periodontal disease.

In the present invention, "treatment" means any act of improving or beneficially modifying the symptoms of a subject suspected of having, or diagnosed with, periodontal disease, or atherosclerosis or Alzheimer's disease caused by periodontal disease.

In one embodiment, the composition of the present invention may prevent or improve periodontal disease by suppressing osteoclastogenesis.

In one embodiment, the composition of the present invention can prevent or improve periodontal disease by suppressing the expression of gingipain, thereby reducing *Porphyromonas gingivalis* colony formation, *Porphyromonas gingivalis* LPS and gingipain in gingival tissue.

In the present invention, the "subject" means any animal, including livestock or mice, that has developed or may develop periodontal disease, or atherosclerosis or Alzheimer's disease caused by periodontal disease, and may be a mammal including a human.

The pharmaceutical composition of the present invention may include an active ingredient alone, or may be provided as a pharmaceutical composition further including one or more pharmaceutically acceptable carriers, excipients or diluents.

The carrier, excipient or diluent that may be included in the pharmaceutical composition of the present invention may include lactose, dextrose, sucrose, dextrin, maltodextrin, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, but is not limited thereto.

The route of administration of the pharmaceutical composition of the present invention may be oral, intravenous, intramuscular, intraarterial, intramedullary, intrathecal, intracardiac, transdermal, subcutaneous, intraperitoneal, intranasal, enteral, topical, sublingual, or rectal administration, but is not limited thereto.

In one embodiment, the composition of the present invention may be administered orally or parenterally.

When the composition of the present invention is administered parenterally, it is preferable to select an administration method such as topical application, intraperitoneal injection, intrarectal injection, subcutaneous injection, intravenous injection, intramuscular injection, or intrathoracic injection, but it is not limited thereto.

The pharmaceutical composition of the present invention may be a solid preparation for oral administration, such as a tablet, pill, powder, granule or capsule.

The pharmaceutical composition of the present invention may be a liquid preparation for oral administration, such as a suspension, a solution, an emulsion or a syrup.

The pharmaceutical composition of the present invention may be a preparation for parenteral administration, such as a sterile aqueous solution, a non-aqueous solution, a suspension, an emulsion, a lyophilized formulation or a suppository.

The present invention provides a kit for use in preventing or treating periodontal disease, or atherosclerosis or Alzheimer's disease caused by periodontal disease, which includes the pharmaceutical composition for preventing or treating periodontal disease, or atherosclerosis or Alzheimer's disease caused by periodontal disease, and instructions describing a method for preventing or treating these diseases.

In one embodiment, a method for preventing or treating periodontal disease, or atherosclerosis or Alzheimer's disease caused by periodontal disease may include administering the pharmaceutical composition of the present invention to a subject who has developed, or is at risk of developing, periodontal disease, or atherosclerosis or Alzheimer's disease caused by periodontal disease.

In the present invention, "administration" means introducing a predetermined substance to a subject by an appropriate method.

In one embodiment, the method for preventing or treating periodontal disease, or atherosclerosis or Alzheimer's disease caused by periodontal disease may include administering a pharmaceutically effective amount of the pharmaceutical composition of the present invention to a subject who has developed, or is at risk of developing, periodontal disease, or atherosclerosis or Alzheimer's disease caused by periodontal disease.

In the present invention, the "pharmaceutically effective amount" refers to an amount sufficient to treat periodontal disease at a reasonable benefit/risk ratio applicable to medical treatment.

The pharmaceutically effective amount of the pharmaceutical composition of the present invention may be determined according to factors such as the severity of the disease, the activity of the pharmaceutical composition, the sensitivity of the subject or patient to the pharmaceutical composition, the time and route of administration, the excretion rate, the duration of treatment, and concomitant medication, as well as other factors well known in the medical field, and may be appropriately selected by a person skilled in the art.

The pharmaceutical composition of the present invention may be administered as a single therapeutic agent or in combination with other therapeutic agents, may be administered sequentially or simultaneously with conventional therapeutic agents, and may be administered in a single or multiple dosage regimen, which can be readily determined by a person skilled in the art.

The present invention provides a health functional food for use in preventing or improving periodontal disease, or atherosclerosis or Alzheimer's disease caused by periodontal disease, which includes a peptide having an amino acid sequence of SEQ ID NO: 1.

The health functional food of the present invention refers to food manufactured and/or processed in various forms to provide functionality beneficial to the human body.

The health functional food of the present invention may be incorporated into various foods or medicines known in the art.

There is no particular limitation on the type of food in which the health functional food of the present invention may be included. For example, the health functional food of the present invention may be included in meat, sausages, bread, chocolate, candies, snacks, confectionery, pizza, ramen, other noodles, gum, dairy products including ice cream, various soups, beverages, teas, drinks, alcoholic beverages, and vitamin complexes.

The health functional food of the present invention encompasses all forms, including functional foods, nutritional supplements, health foods, and food additives, and may be manufactured in various forms according to conventional methods known in the art. For example, the health food may be manufactured in the form of a liquid drink for oral intake, or may be granulated, encapsulated, tableted into spherical pills, or powdered for consumption. It may also be manufactured in the form of a powder, capsule, soft capsule, tablet, gum, or adhesive-type liquid composition. In addition, the functional food may include beverages (including alcoholic beverages); fruits and processed products thereof (e.g., canned fruits, bottled fruits, jams, marmalades, etc.); fish, meat and processed products thereof (e.g., ham, sausages, corned beef, etc.); breads and noodles (e.g., udon, buckwheat noodles, ramen, spaghetti, macaroni, etc.); fruit juices, various drinks, cookies, taffy, dairy products (e.g., butter, cheese, etc.); edible vegetable oils, margarine, vegetable proteins, retort foods, frozen foods, herbal decoctions, and seasonings (e.g., soybean paste, soy sauce, sauces, etc.).

The health functional food of the present invention may further include ingredients commonly added during food manufacture, as long as it does not deviate from the ultimate purpose of the present invention, and may further include, for example, proteins, carbohydrates, fats, other nutrients, seasonings, and flavoring agents.

The health functional food of the present invention may additionally contain various nutritional supplements, vitamins, electrolytes, flavoring agents, coloring agents, pectic acids and their salts, alginic acid and its salts, organic acids, protective colloid thickeners, pH regulators, stabilizers, preservatives, glycerin, alcohol, carbon dioxide agents used in carbonated beverages, etc.

The health functional food of the present invention may contain fruit pulp for the production of natural fruit juice, fruit juice beverages, and vegetable beverages. These ingredients may be used independently or in combination.

Hereinafter, the present invention will be described in detail by way of examples in order to specifically explain the present invention. However, the following examples are provided only to facilitate understanding of the present invention, and the content of the present invention is not limited thereto.

### Example

### 1. Experimental method

### 1.1 Synthesis of GV1001

A peptide consisting of 16 amino acids, having the structure according to Formula 1 below and the amino acid sequence of SEQ ID NO: 1 (GV1001) derived from human telomerase, was synthesized.

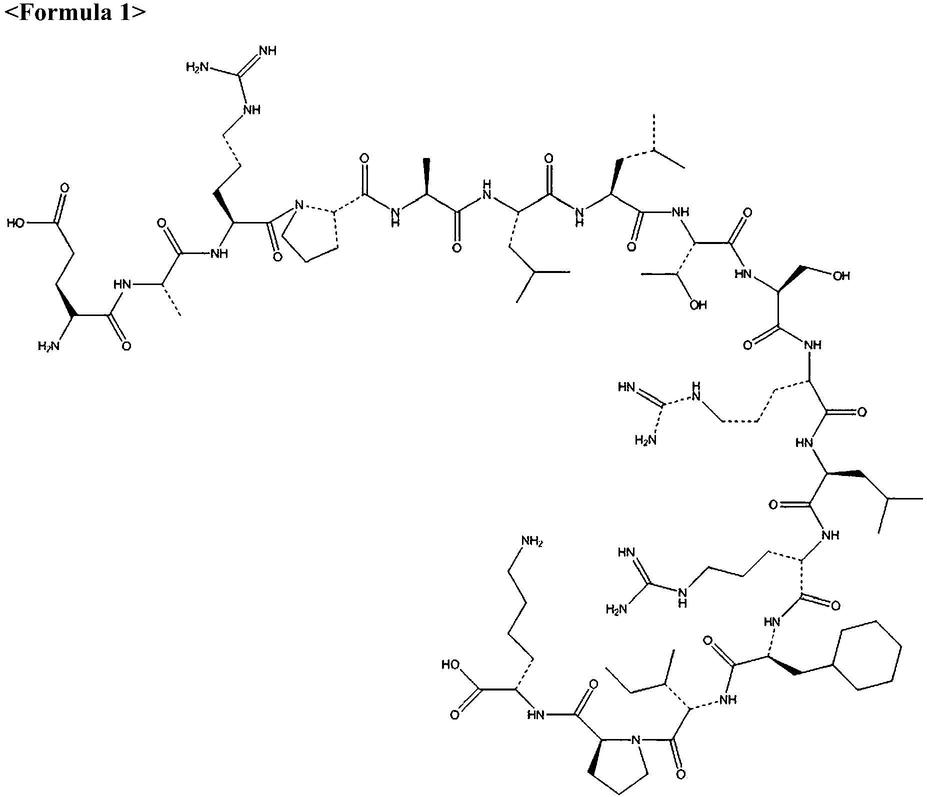

Peptide GV1001 of SEQ ID NO: 1 was prepared according to a conventional solid-phase peptide synthesis method. Specifically, the peptides were synthesized by coupling amino acids sequentially from the C-terminus through Fmoc solid phase peptide synthesis (SPPS) using ASP48S (Peptron, Inc., Daejeon, Korea). The peptides were used, in which the first amino acid at the C-terminus was attached to the resin as follows. For example:
NH₂ -Lys(Boc)-2-chloro-Trityl Resin
NH₂ -Ala-2-chloro-Trityl Resin
NH₂ -Arg(Pbf)-2-chloro-Trityl Resin

All amino acid starting materials used for peptide synthesis were protected at the N-terminus with Fmoc, and all residues were protected with Trt, Boc, t-Bu (t-butylester), Pbf (2,2,4,6,7-pentamethyl dihydro-benzofuran-5-sulfonyl), etc., which are removable under acidic conditions. For example:
Fmoc-Ala-OH, Fmoc-Arg(Pbf)-OH, Fmoc-Glu(OtBu)-OH, Fmoc-Pro-OH, Fmoc-Leu-OH, Fmoc-Ile-OH, Fmoc-Phe-OH, Fmoc-Ser(tBu)-OH, Fmoc-Thr(tBu)-OH, Fmoc-Lys(Boc)-OH, Fmoc-Gln(Trt)-OH, Fmoc-Trp(Boc)-OH, Fmoc-Met-OH, Fmoc-Asn(Trt)-OH, Fmoc-Tyr(tBu)-OH, Fmoc-Ahx-OH, Trt-Mercaptoacetic acid.

As coupling reagents, HBTU [2-(1H-Benzotriazole-1-yl)-1,1,3,3-tetramethylaminium hexafluorophosphate]/ HOBt [N-Hydroxybenzotriazole]/NMM [4-Methylmorpholine] were used. Piperidine in DMF (20%) was used for Fmoc removal. A cleavage cocktail [TFA (trifluoroacetic acid)/TIS (triisopropylsilane)/EDT (ethanedithiol)/H₂O = 92.5/2.5/2.5] was used to cleave the synthesized peptide from the resin and remove protecting groups of the residue.

Using a state in which the starting amino acid having a protecting group bonded thereto was bound to a solid support, a process of reacting each corresponding amino acid, washing with a solvent, and then deprotecting was repeated to synthesize each peptide. The synthesized peptide was cleaved from the resin, purified by HPLC, followed by confirming whether it was synthesized by MS, and then freeze-dried.

The specific synthesis process of GV1001 is described as follows.

### 1) Coupling

NH₂-Lys(Boc)-2-chloro-Trityl resin was added with protected amino acid (8 equivalents) and coupling reagents HBTU (8 equivalents)/HOBt (8 equivalents)/NMM (16 equivalents) dissolved in DMF, followed by reaction at room temperature for 2 hours, and washing with DMF, MeOH, and DMF in this order.

### 2) Fmoc deprotection

Piperidine in DMF (20%) was added and reacted twice for 5 minutes at room temperature, and then washed with DMF, MeOH, and DMF in this order.
3) A peptide backbone was constructed by repeatedly performing reactions 1 and 2.
4) Cleavage: A cleavage cocktail was added to the peptide resin after completion of the synthesis to separate the peptide from the resin.
5) Cooled diethyl ether was added to the obtained mixture, and the resulting peptide was precipitated by centrifugation.
6) After purification by Prep-HPLC, the molecular weight was confirmed by LC/MS, and the product was frozen to manufacture powder.

### 1.2 Animal model production and experimental protocol

An animal model was created using *ApoE⁻*/*⁻* mice (C57BL/6 background, male, 4 weeks old).

Animal models were fed with a high-fat diet (HFD), and a 6-0 silk ligature was placed around the maxillary second molar to create a gingival pocket using the ligature placement method for 1 week, followed by treatment with *Porphyromonas gingivalis* (*Pg*) or PBS for 5 weeks. The animal model groups used in the experiment are shown in Table 1 below.

**[TABLE 1]**

| **Group** | **Model induction** | **Drug** | **Dose (mg/kg)** | **Number** | **Administration route** | **Administration time** |
|---|---|---|---|---|---|---|
| **Sham** | PBS | PBS | - | 10 | i.p. | Start: 1 week before ligation |
| **Drug only group** | PBS | GV1001 | 2 | 10 | i.p. | |
| | | | | | | Number of sessions: 3 |
| **Control** | *Pg* | PBS | - | 10 | i.p. | |
| **Drug treatment group** | application | GV1001 | 2 | 10 | i.p | times/week for 10 weeks |

### 1.3 Evaluation items

### 1) Evaluation of efficacy related to periodontitis

The depth of the periodontal pocket, the expression level of inflammatory cytokines in the gingival tissue (mRNA level, IF staining), the aggregation of *Porphyromonas gingivalis, Porphyromonas gingivalis* LPS, Kgp and RgpB in the gingival tissue, the amount of alveolar bone loss, and the number of osteoclasts in the alveolar bone were determined. Further, the effect on osteoclastogenesis was confirmed in an *in vitro* test.

### 2) Evaluation of efficacy related to atherosclerosis

The expression levels of inflammatory cytokines in serum and arterial tissue, the aggregation of *Porphyromonas gingivalis*, *Porphyromonas gingivalis* LPS, Kgp and RgpB in arterial tissue, changes in cholesterol levels in serum, and lipid deposition (atherosclerosis) in the arterial wall were determined. *In vitro,* inhibition of EndMT and inhibition of macrophage uptake of dil-ox-LDL were evaluated. Further, the CD47 expression levels were determined in the aortic root *in vivo* and *in vitro.*

### 3) Evaluation of efficacy related to Alzheimer's disease (AD)

The expression levels of inflammatory cytokines, the aggregation of *Porphyromonas gingivalis, Porphyromonas gingivalis* LPS and Kgp, RgpB, and the accumulation of Aβ₄₂ and p-Tau in brain tissue were confirmed.

### 2. Experimental results

### 2.1 Effect of GV1001 in periodontitis

### 1) Inflammation

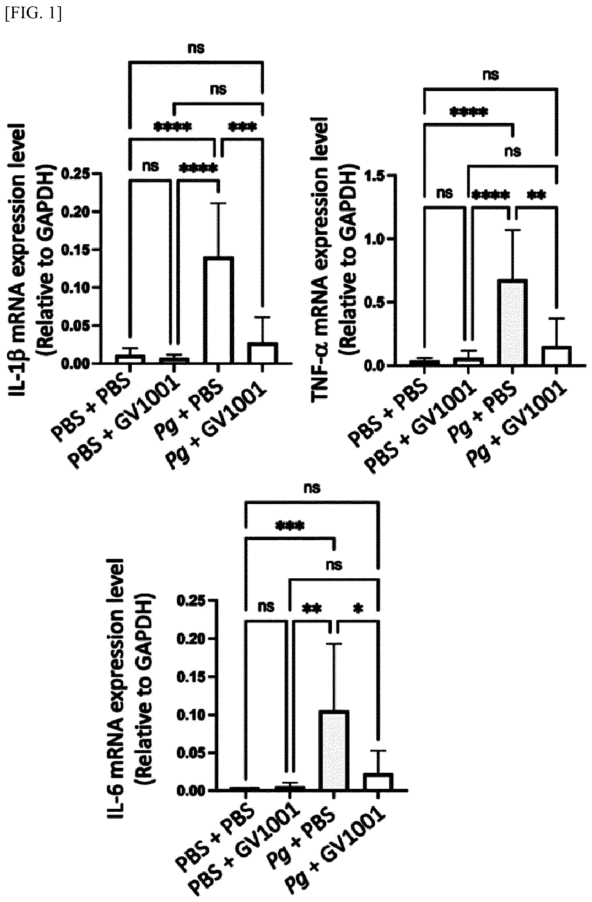

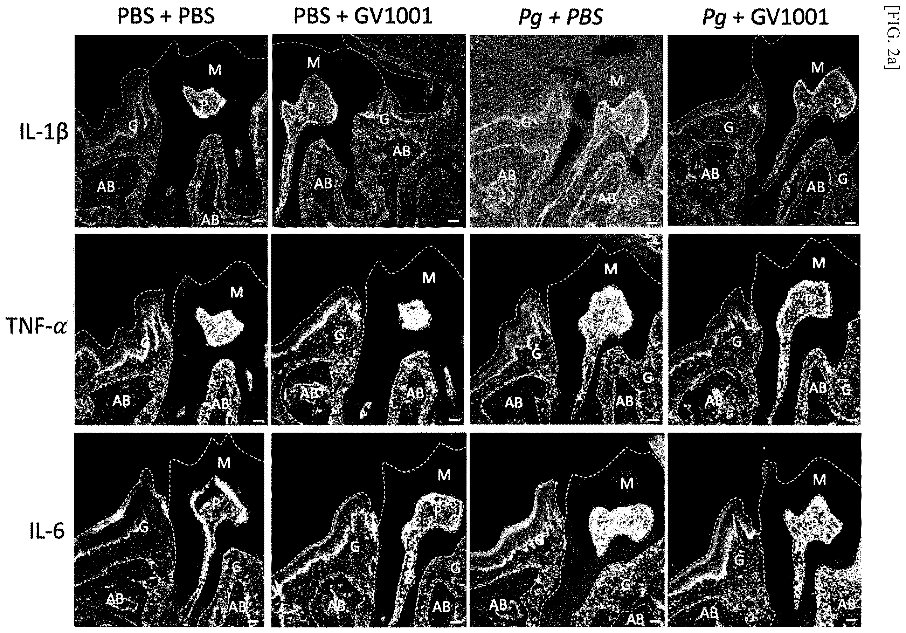

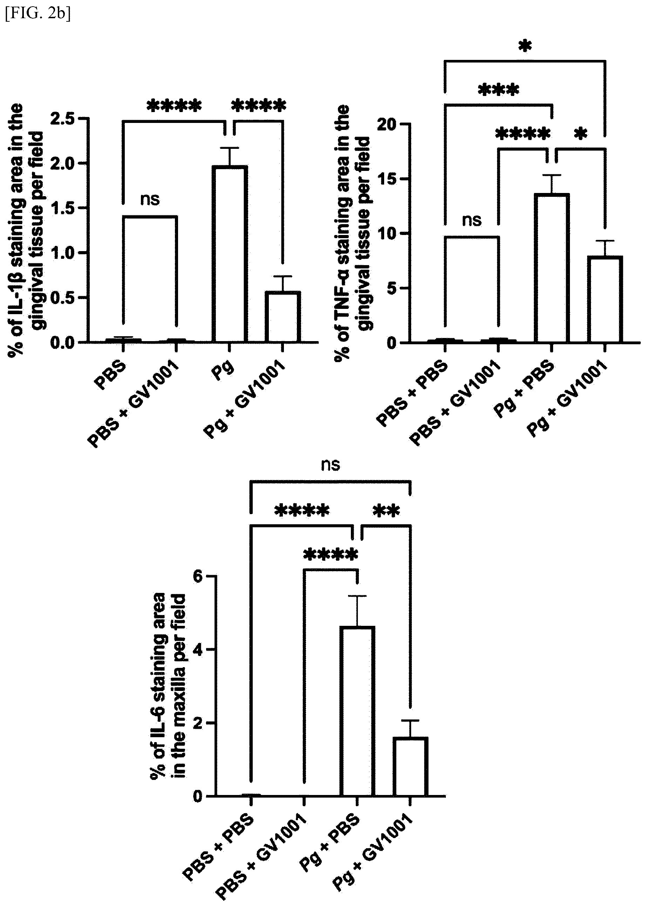

To confirm changes in inflammatory cytokines (IL-1β, TNF-α, IL-6) in periodontal tissue, cytokine mRNA levels (FIG. 1) and cytokine expression levels (FIG. 2) were measured by fluorescent staining.
- Control group: The amount of cytokines increased in the periodontitis-induced model (control group).
- GV1001 administration group: Cytokine mRNA levels were significantly reduced by GV1001 administration, and a decrease in cytokine expression was confirmed by fluorescence staining.

### 2) Porphyromonas gingivalis colonies and gingipain

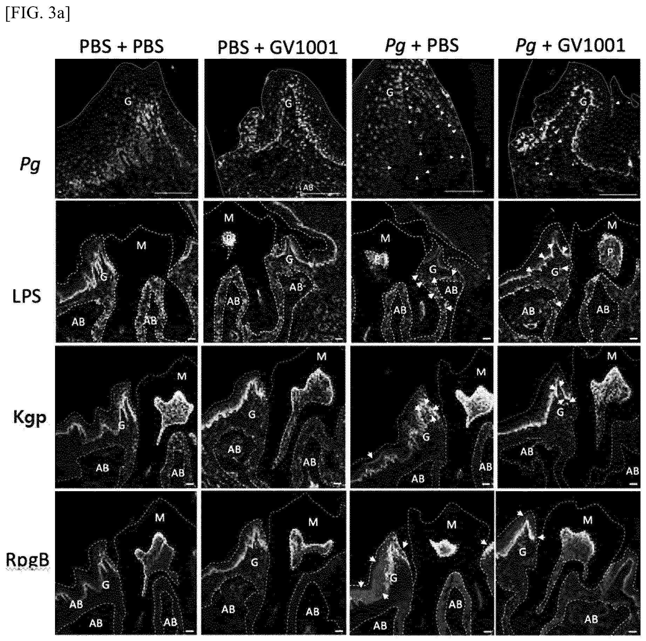

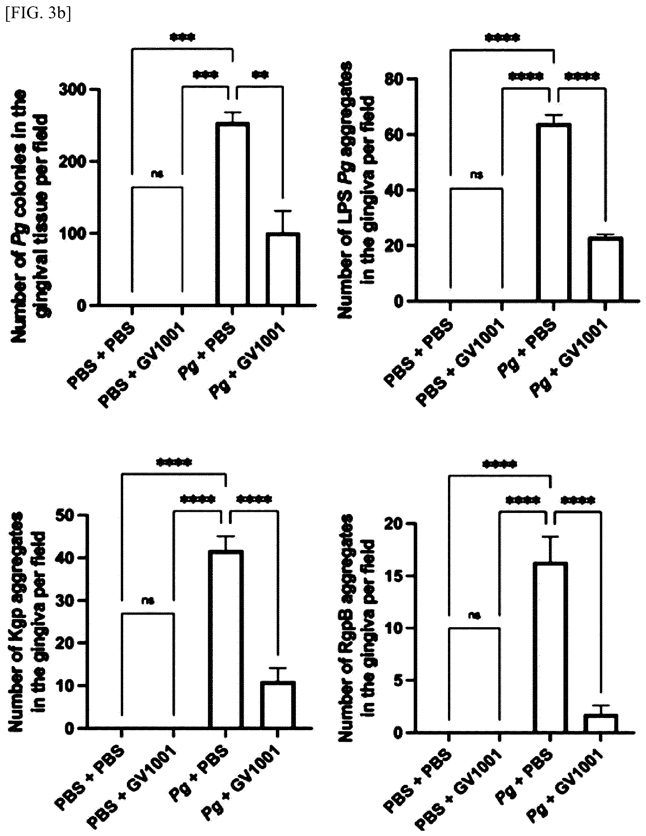

The number of *Porphyromonas gingivalis* colonies and *Porphyromonas gingivalis* LPS were determined in gingival tissue (FIG. 3), and changes in aggregates of gingipain (Kgp, RgpB) were confirmed.
- Control group: In the periodontitis-induced model (control group), the number of *Porphyromonas gingivalis* colonies, *Porphyromonas gingivalis* LPS and gingipain in the gingival tissue were increased.
- GV 1001 administration group: The number of *Porphyromonas gingivalis* colonies, *Porphyromonas gingivalis* LPS and gingipain in the gingival tissue were significantly reduced.

### 3) Osteoclasts

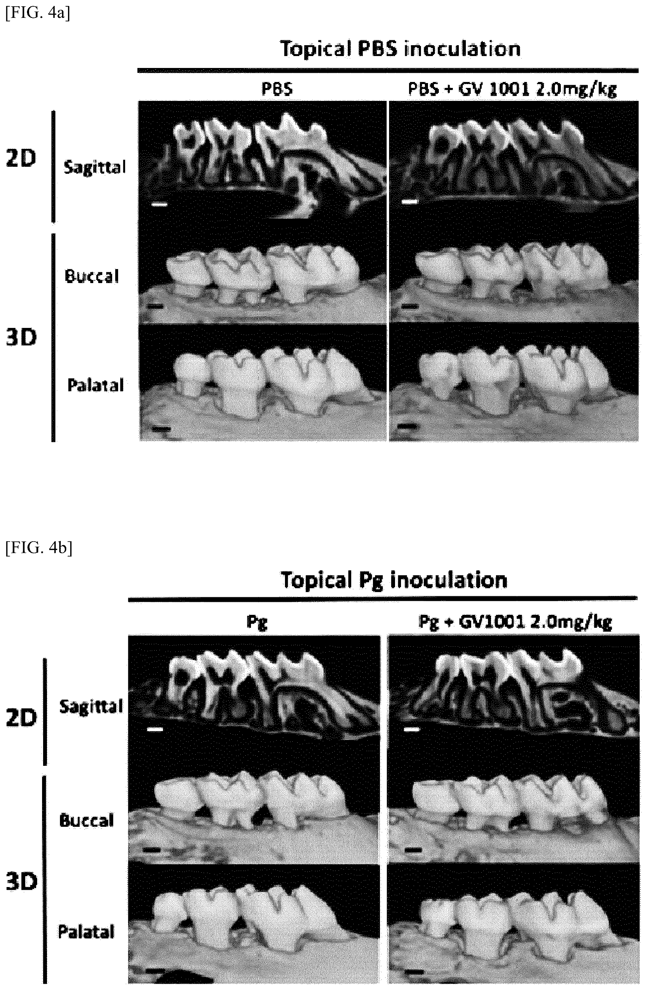

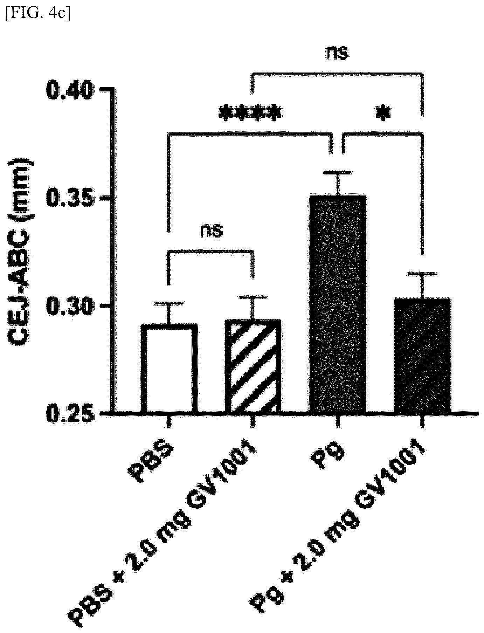

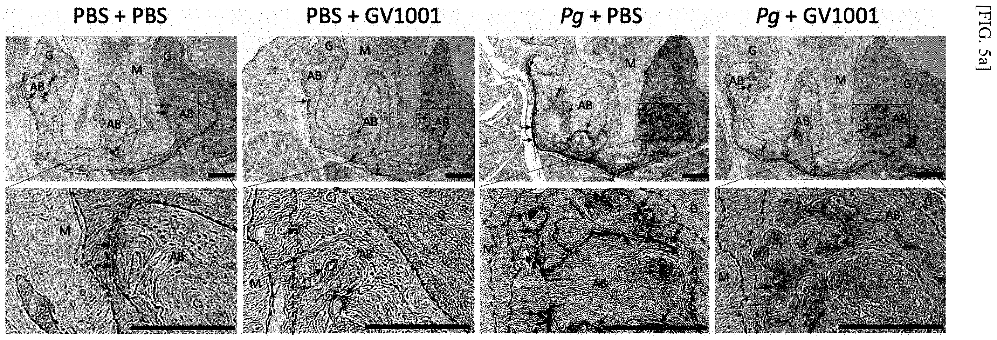

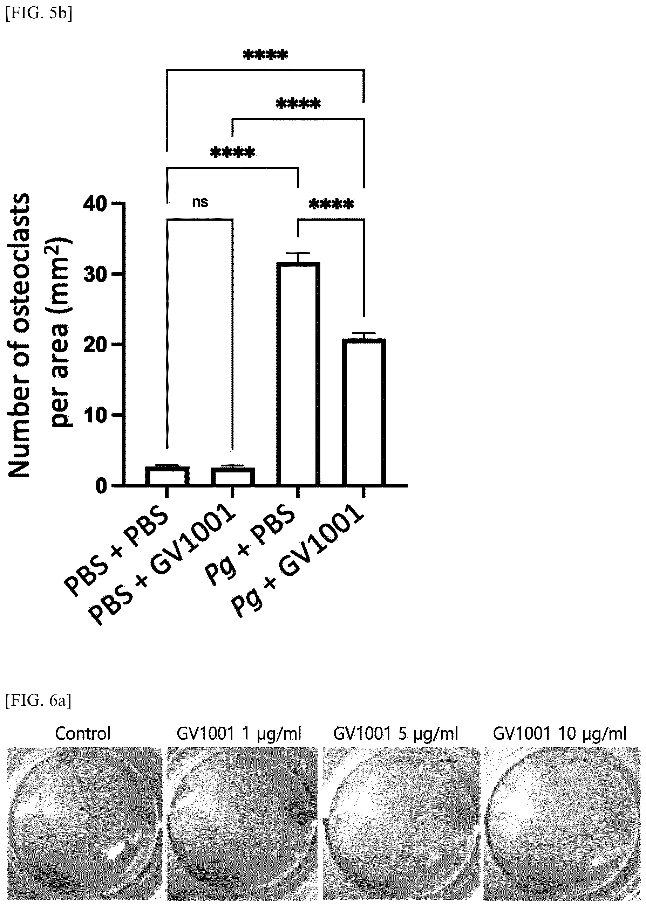

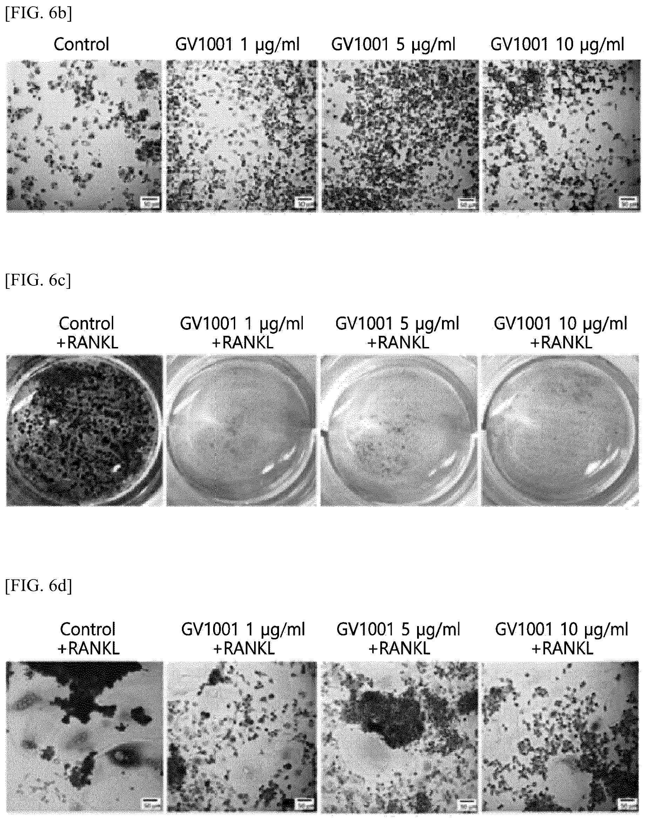

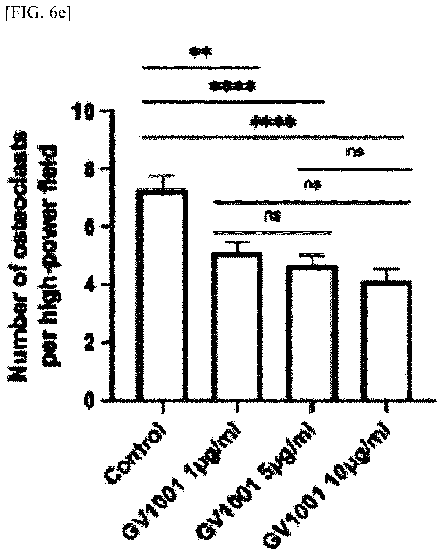

To confirm the efficacy of GV1001 on alveolar bone loss, µCT was used to determine alveolar bone loss in a periodontitis-induced animal model (FIG. 4), and the number of osteoclasts in the alveolar bone was determined by TRAP staining (FIG. 5). Further, osteoclastogenesis, i.e., the degree of differentiation of monocytes into osteoclasts, was measured *in vitro* (FIG. 6).
- Control group: In the periodontitis-induced model (control group), osteoclast formation in the alveolar bone increased and alveolar bone loss was confirmed.
- GV1001 administration group: It was confirmed that osteoclastogenesis was significantly inhibited and alveolar bone loss was significantly reduced by GV1001 administration. After inducing the macrophage cell line (Raw264.7) into osteoclasts, osteoclastogenesis was significantly inhibited by GV1001.

### 4) Systemic inflammation

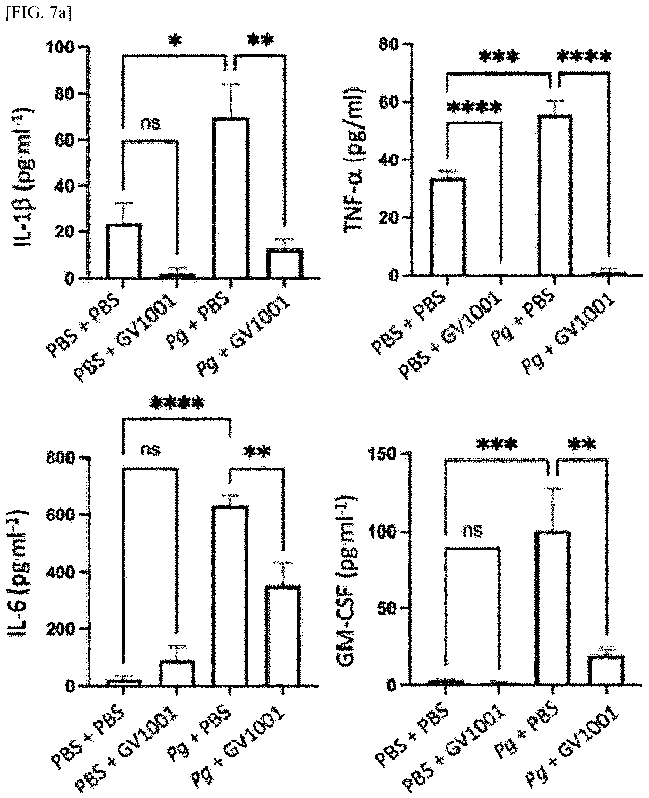

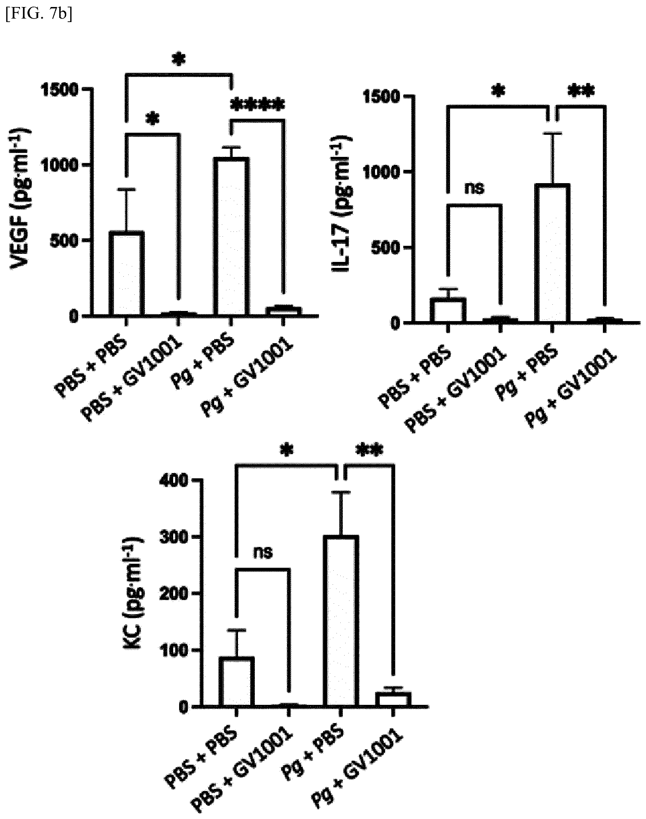

To determine the level of progression of systemic inflammation caused by periodontitis, the amount of inflammatory cytokines (IL-1β, TNF-α, IL-6, GM-CSF, VEGF, IL-17, KC) was measured in the serum of the periodontitis-induced model (FIG. 7).
- Control group: All cytokines were increased in the serum of the periodontitis-induced model (control group).
- GV1001 administration group: All cytokines in the serum were significantly suppressed by GV1001 administration.

### 2.2 Effect of GV1001 in periodontitis-related atherosclerosis

### 1) Vascular inflammation

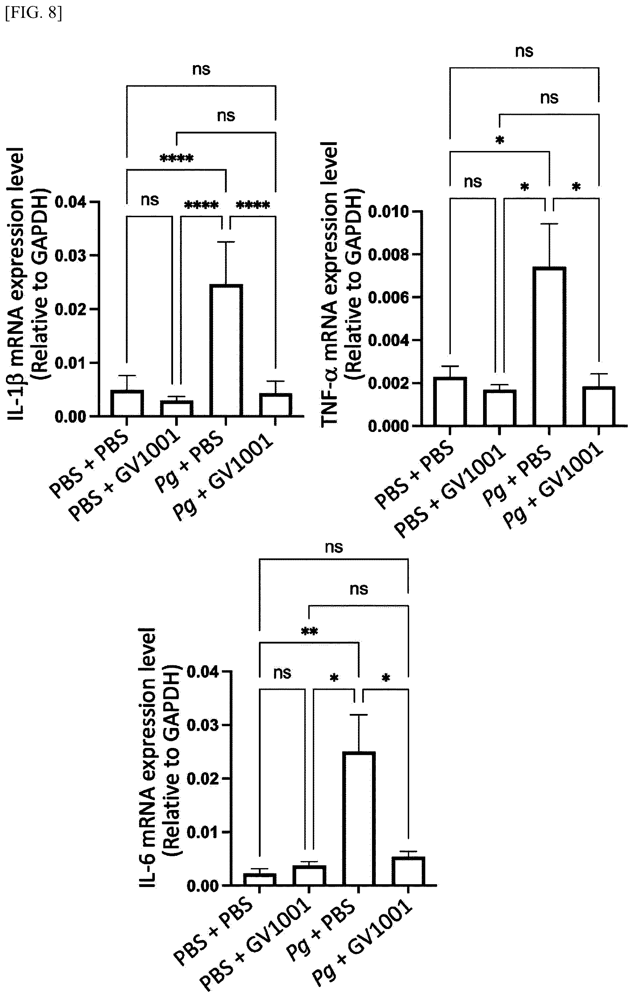

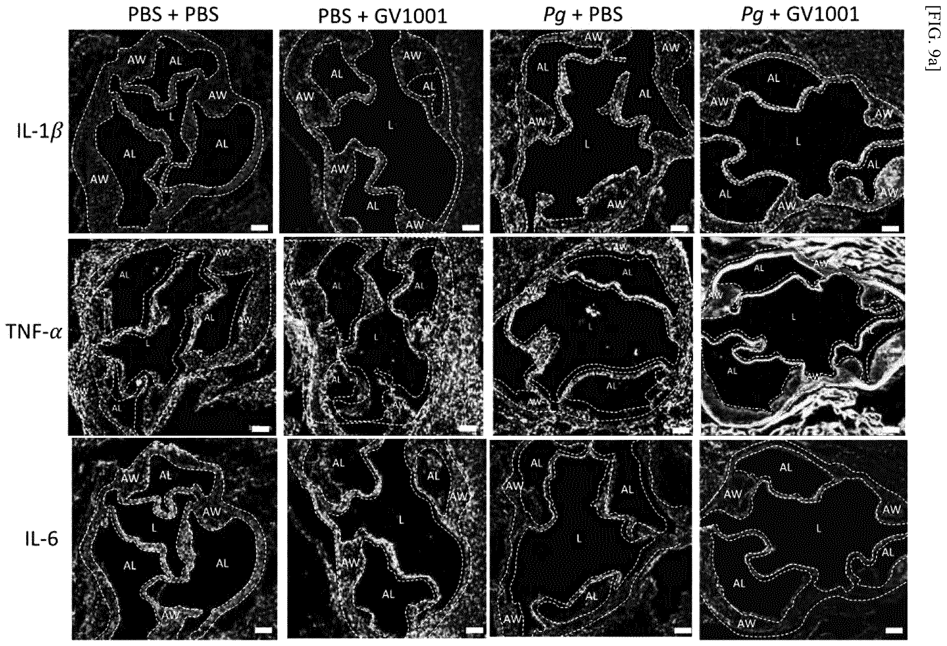

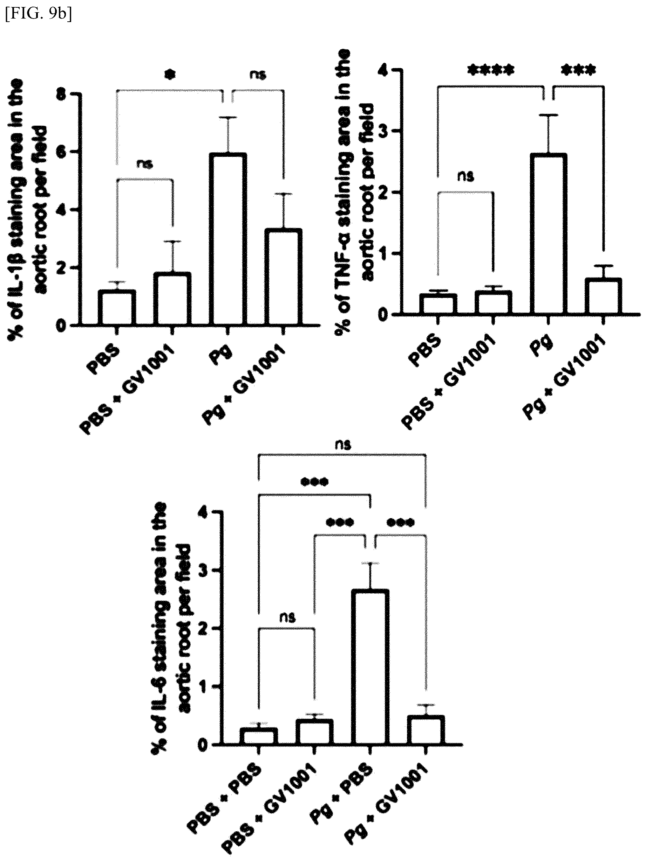

Since it is well known that atherosclerosis is induced by vascular inflammation, a change in the amount of inflammatory cytokines (IL-1β, TNF-α, IL-6) in arterial tissue was measured to confirm the possibility that periodontitis progresses to atherosclerosis. To this end, cytokine mRNA levels (FIG. 8) and cytokine expression levels (FIG. 9) were measured by fluorescent staining.
- Control group: In the periodontitis-induced model (control group), the amount of cytokines in the arteries was increased.
- GV1001 administration group: Cytokine mRNA levels were significantly reduced by GV1001 administration, and a decrease in cytokine expression was confirmed by fluorescence staining.

### 2) Porphyromonas gingivalis colonies and gingipain

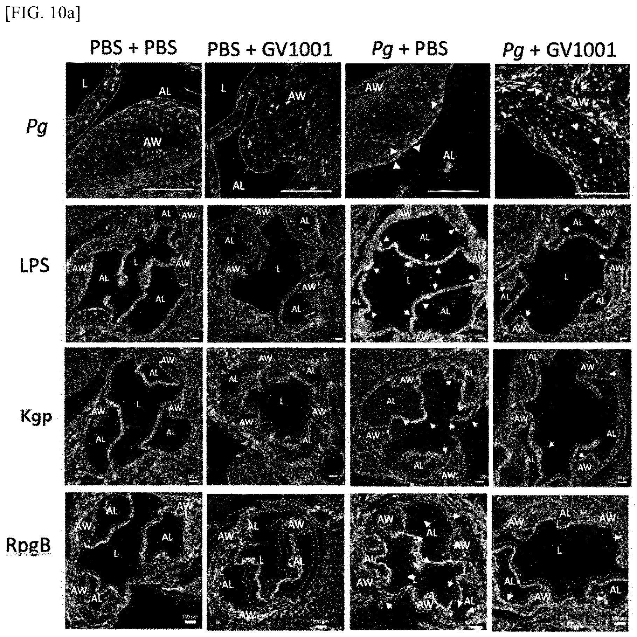

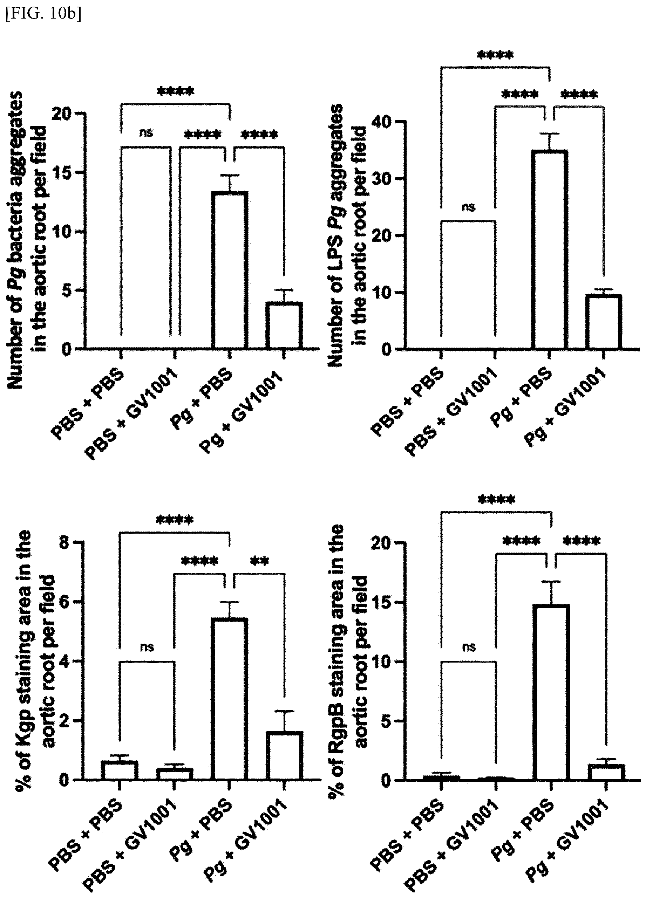

The number of *Porphyromonas gingivalis* colonies and changes in the aggregates of *Porphyromonas gingivalis* LPS and gingipain (Kgp, RgpB) were confirmed in the arterial wall (FIG. 10).
- Control group: In the periodontitis-induced model (control group), the number of *Porphyromonas gingivalis* colonies, and *Porphyromonas gingivalis* LPS and gingipain in the arterial wall were increased.
- GV1001 administration group: It was confirmed that the number of *Porphyromonas gingivalis* colonies, and *Porphyromonas gingivalis* LPS and gingipain in the arterial wall were significantly reduced.

### 3) Cholesterol

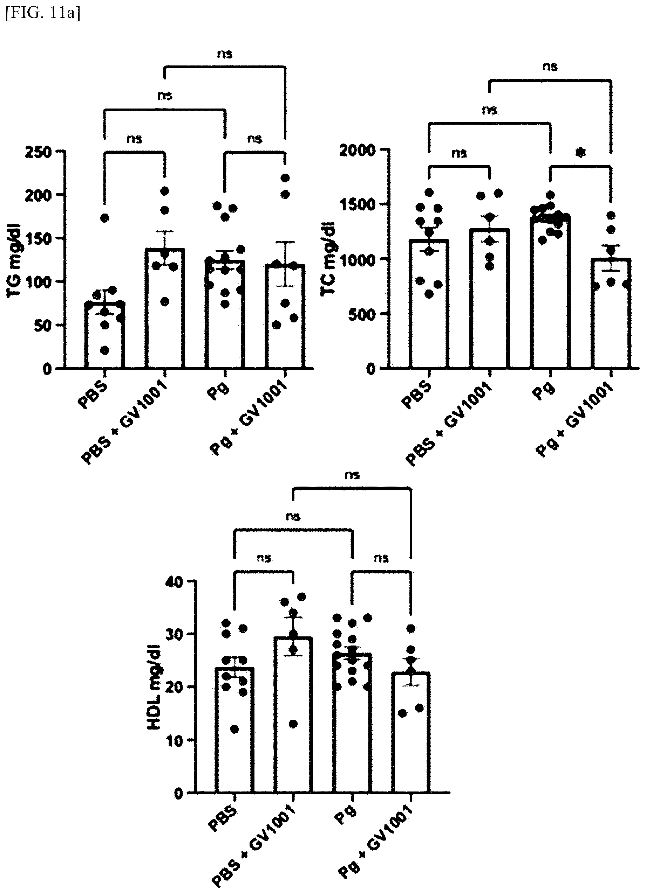

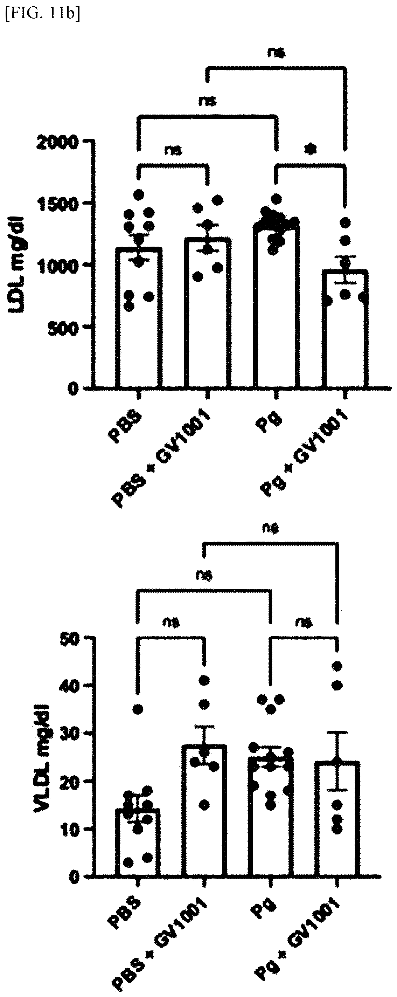

To confirm the change in cholesterol due to chronic inflammation, the levels of serum triglycerides (TG), total cholesterol (TC), high-density lipoprotein (HDL), low-density lipoprotein (LDL), and very low-density lipoprotein (VLDL) were measured (FIG. 11).
- Control group: In the periodontitis-induced model (control group), the levels of serum TG, TC, HDL, LDL and VLDL were increased.
- GV1001 administration group: The levels of TC and LDL in serum were significantly reduced by GV1001 administration.

### 4) Lipid deposition

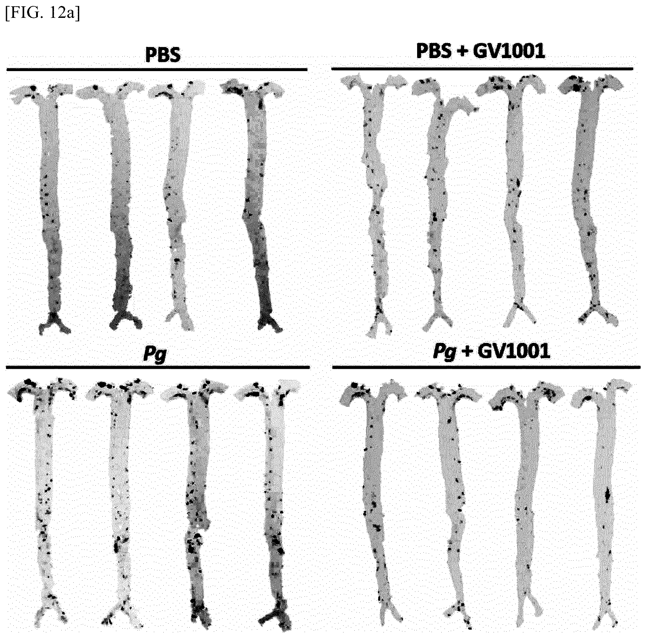

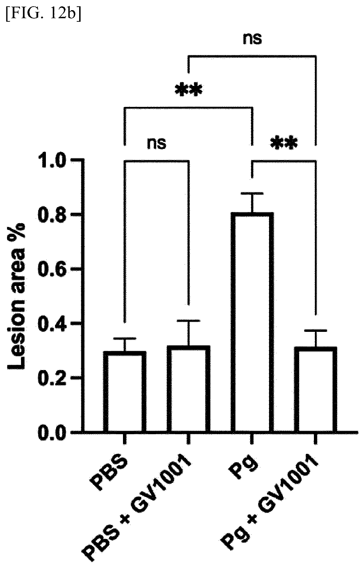

To verify the induction of atherosclerosis by periodontitis, the level of lipid deposition in the wall of the aorta-iliac bifurcation was confirmed using Sudan IV staining and Oil Red O staining (FIG. 12).
- Control group: In the periodontitis-induced model (control group), intravascular lipid deposition was increased.
- GV1001 administration group: Intravascular lipid deposition was significantly suppressed by GV1001 administration.

### 5) Phenotypic changes in endothelial cells

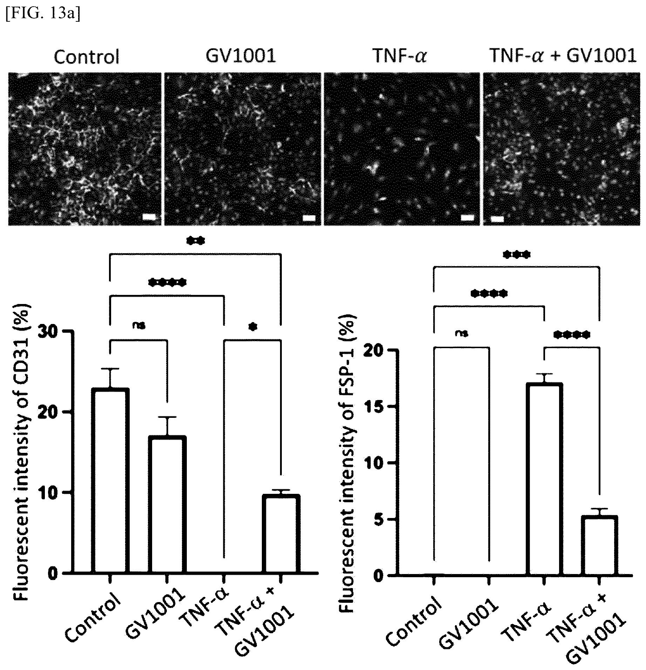

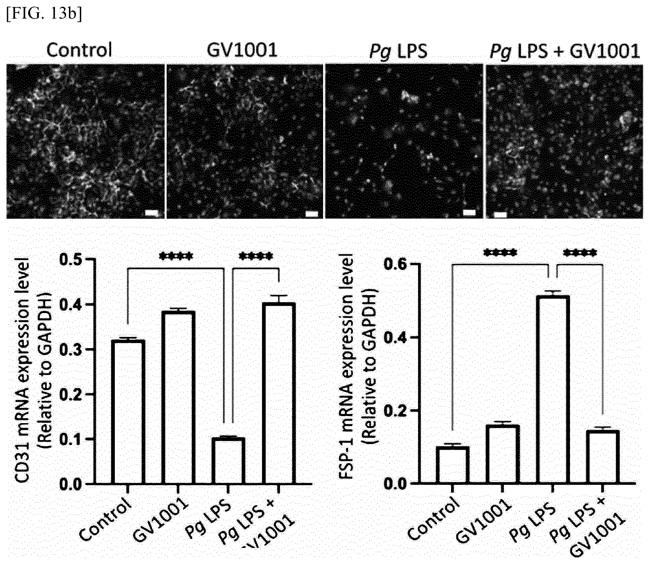

It is known that when endothelial cells (ECs) change their phenotype into mesenchymal cells (MCs) through endothelial-to-mesenchymal transition (EndMT), it induces various vascular diseases (atherosclerosis, pulmonary hypertension, fibrosis, etc.). TNF-α is known as a cytokine involved in this process. In the present invention, it was confirmed that when human umbilical vein endothelial cells (HUVECs) were stimulated with TNF-α and *Porphyromonas gingivalis* LPS, ECs (marker; CD31) were decreased while MCs (marker; FSP-1) were increased due to EndMT (FIG. 13).
- Control group: EndMT of HUVECs was confirmed to progress by TNF-α and *Porphyromonas gingivalis* LPS.
- GV1001 administration group: The EndMT progression of HUVECs was confirmed to be inhibited by GV1001. As a result, it was confirmed that GV1001 can inhibit the development of atherosclerosis induced by periodontitis by suppressing the phenotypic changes of ECs.

### 6) LDL uptake and foam cell formation

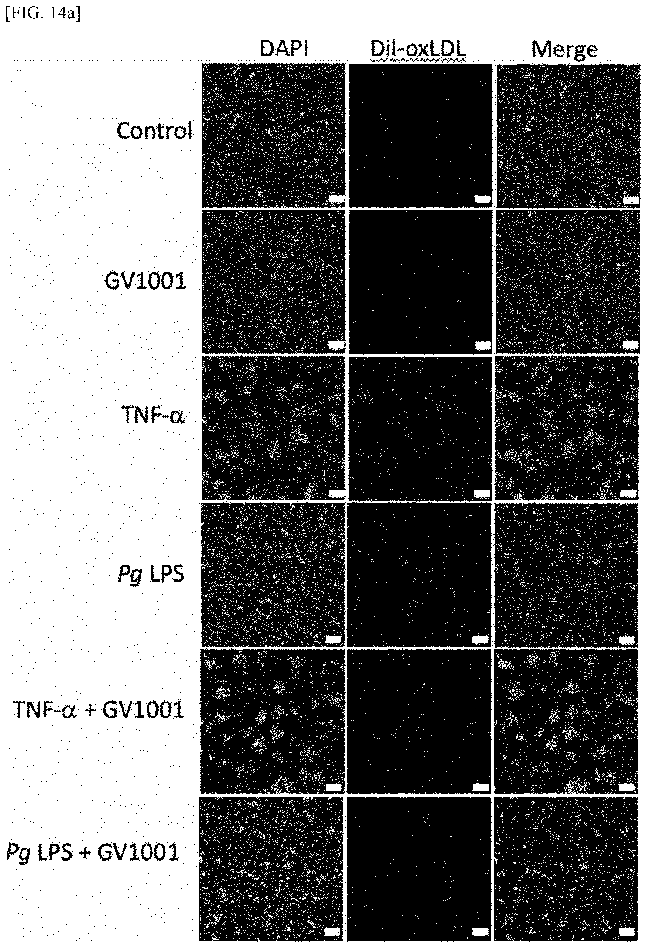

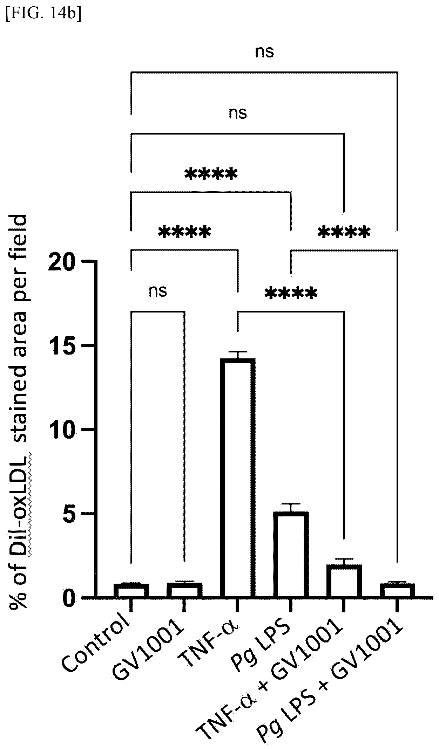

It is known that the formation of foam cells by macrophages in response to LDL and TNF-α is an important process in atherosclerosis. In the present invention, ox-LDL uptake by TNF-α and *Porphyromonas gingivalis* LPS was evaluated in macrophages differentiated from THP-1 (human monocyte cell line) cells (FIG. 14).
- Control group: ox-LDL uptake was increased by TNF-α and *Porphyromonas gingivalis* LPS.
- GV1001 administration group: ox-LDL uptake was significantly inhibited by GV1001. As a result, this means that the inhibition of atherogenesis by GV1001 is partly due to its inhibitory effect on foam cell formation.

### 7) CD47 (Cluster of differentiation 47)

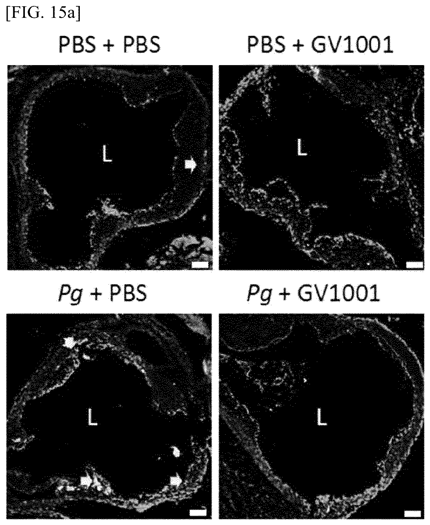

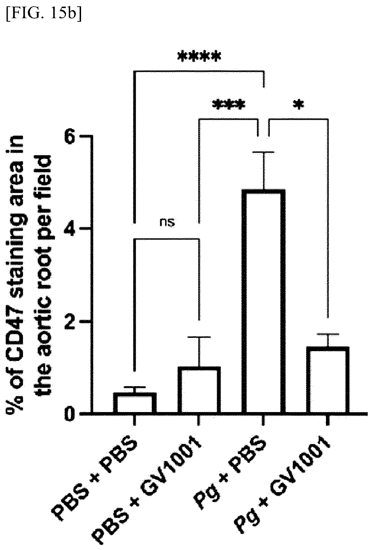

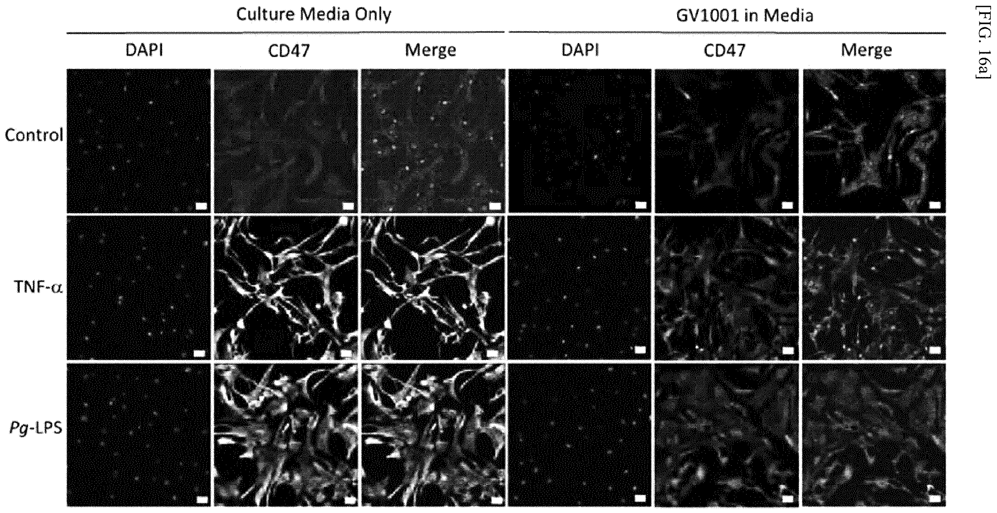

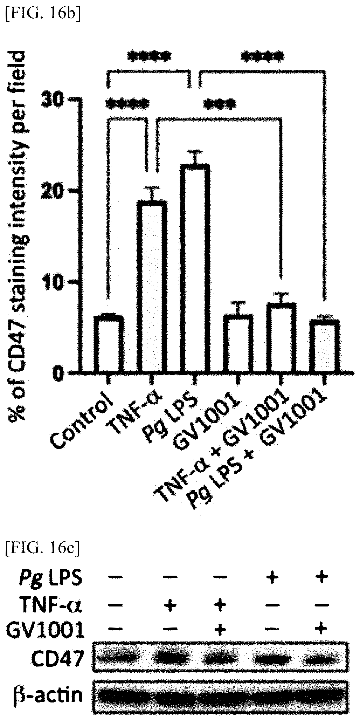

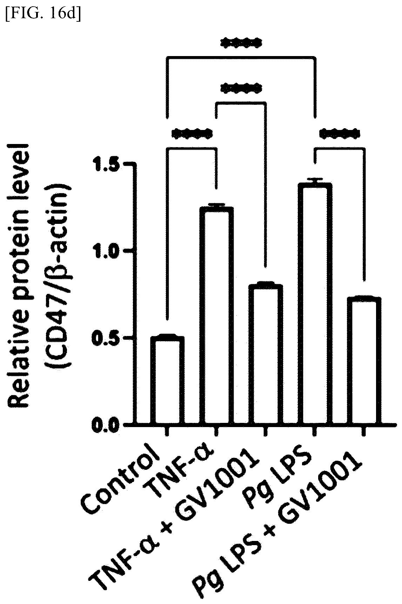

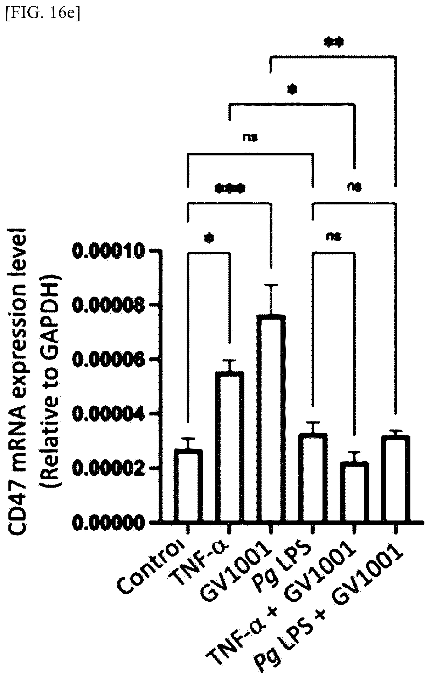

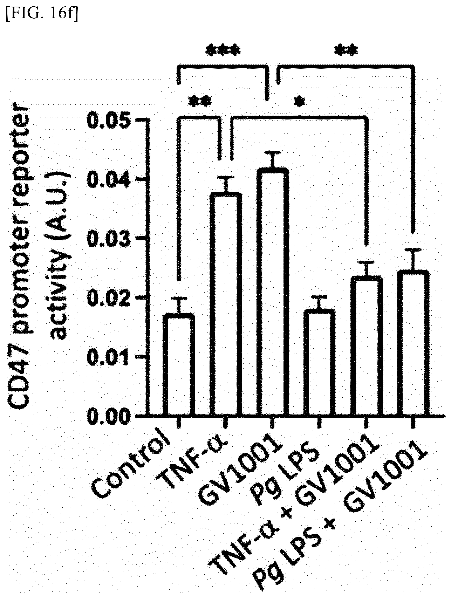

CD47, a representative anti-phagocytic factor, is known to interfere with the removal of cancer cells by macrophages, and overexpression of CD47 is known to cause atherosclerosis. In the present invention, the expression level of CD47 in the aortic root was confirmed in an animal model of periodontitis induction (FIG. 15). Further, the expression level of CD47 was confirmed using human coronary artery smooth muscle cells (HCASMC) at the *in vitro* level (FIG. 16).
- Control group: In the periodontitis-induced model (control group), it was confirmed that CD47 in the aortic root was increased, while, in HCASMC cells, CD47 was increased by TNF-α and *Porphyromonas gingivalis* LPS treatment.
- GV1001 administration group: In an animal model of periodontitis induction, the expression level of CD47 in the aortic root was significantly reduced by administration of GV1001. Further, in an *in vitro* HCASMC cell line study, it was confirmed that the expression level of CD47 was significantly reduced by treatment with TNF-α and *Porphyromonas gingivalis* LPS.

### 2.3 Effect of GV1001 in Alzheimer's disease associated with periodontitis

### 1) Inflammation

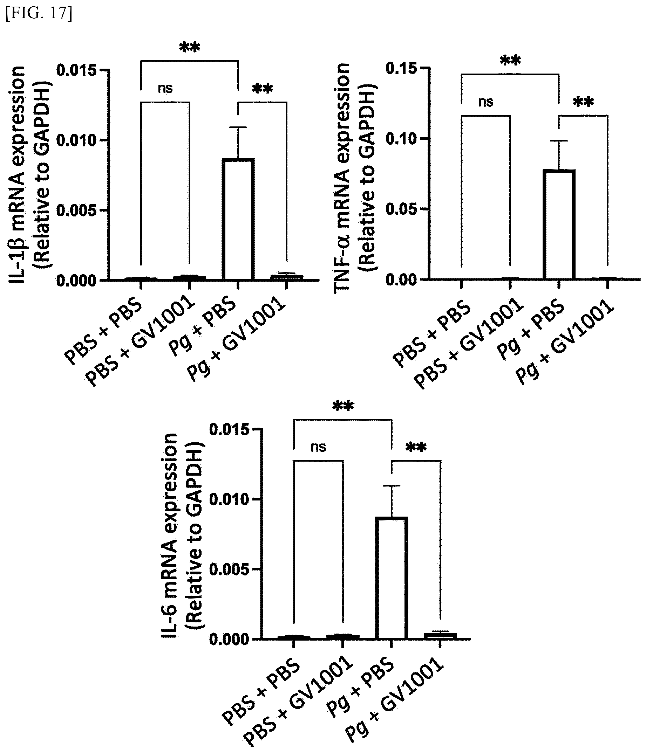

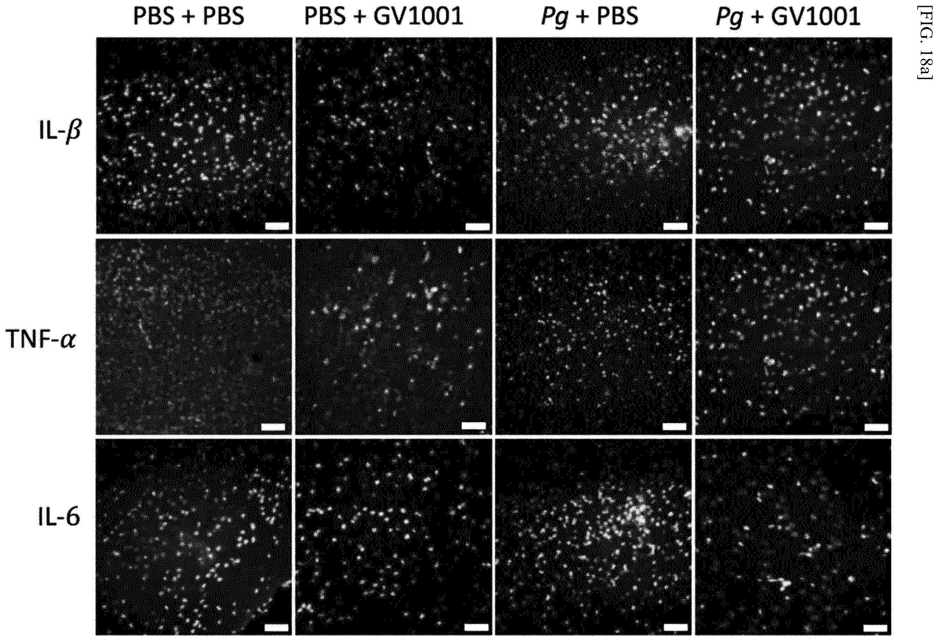

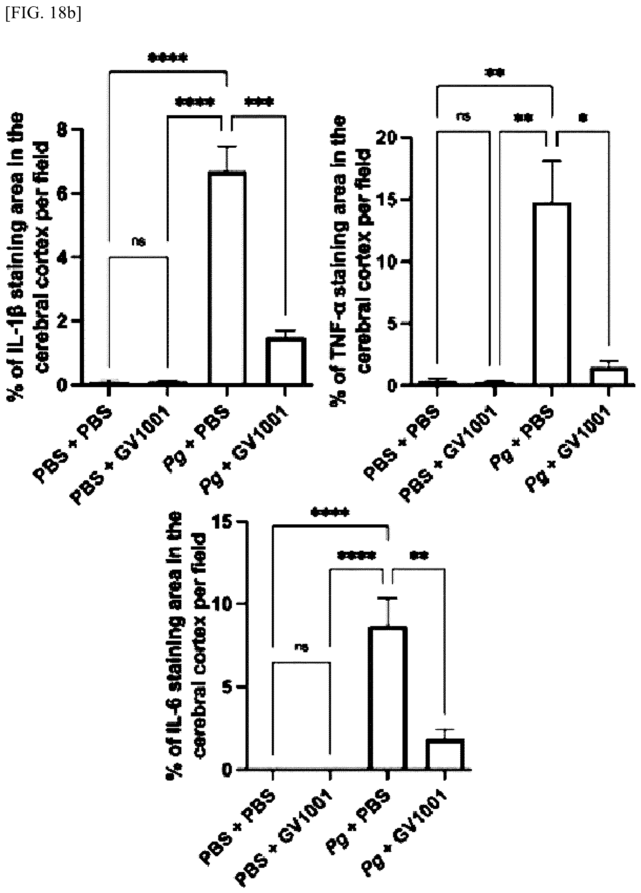

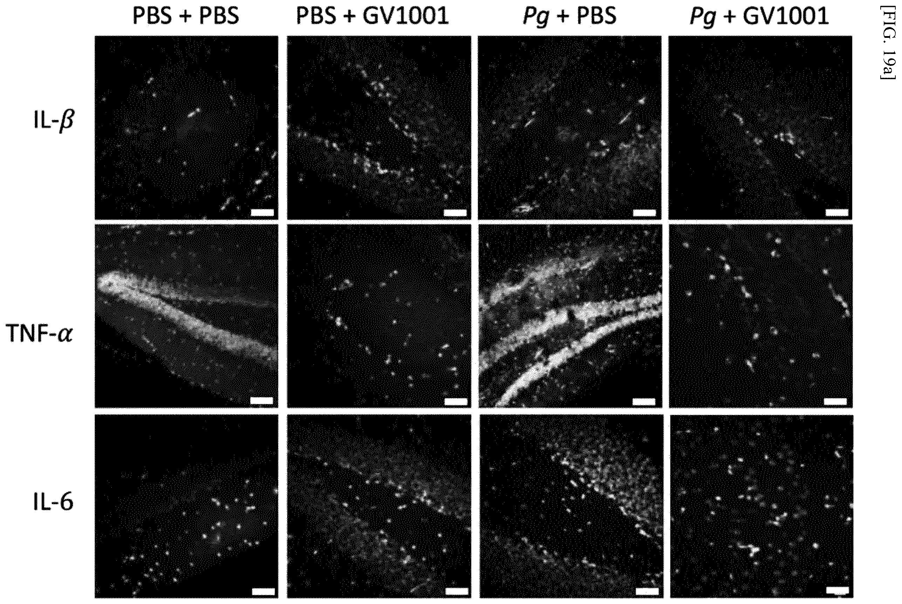

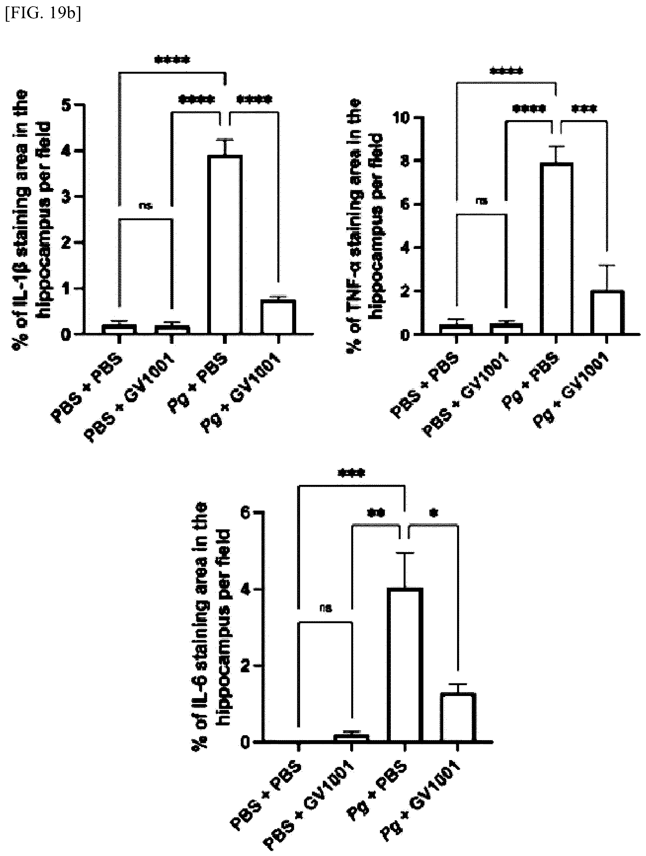

Since Alzheimer's disease patients exhibit neuroinflammation characterized by changes in inflammatory cytokines in the brain, to confirm the possibility that periodontitis may progress to Alzheimer's, the levels of cytokine mRNA were measured to confirm changes in the amount of inflammatory cytokines (IL-1β, TNF-α, IL-6) in brain tissue (FIG. 17), while the level of cytokine expression was measured by fluorescent staining (FIGS. 18 and 19).
- Control group: In the periodontitis-induced model (control group), the amount of cytokines in brain tissue was increased.
- GV1001 administration group: Cytokine mRNA levels were significantly reduced by GV1001 administration, and a decrease in cytokine expression was confirmed by fluorescence staining.

### 2) Porphyromonas gingivalis colonies and gingipain

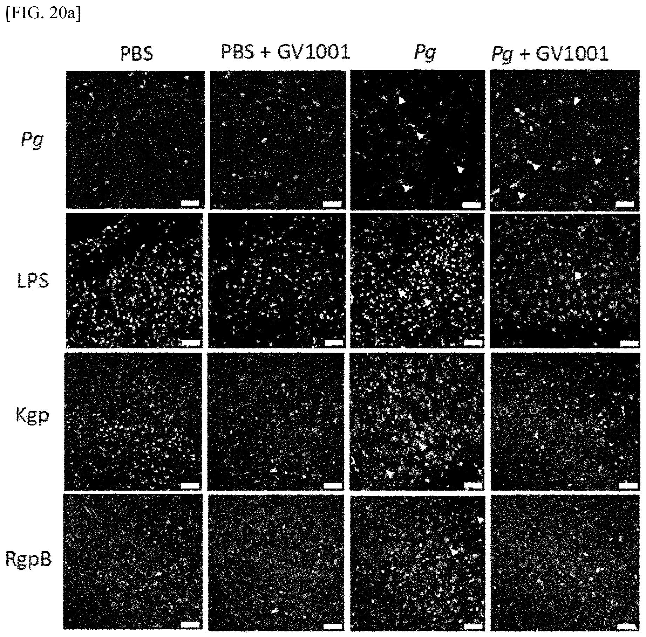

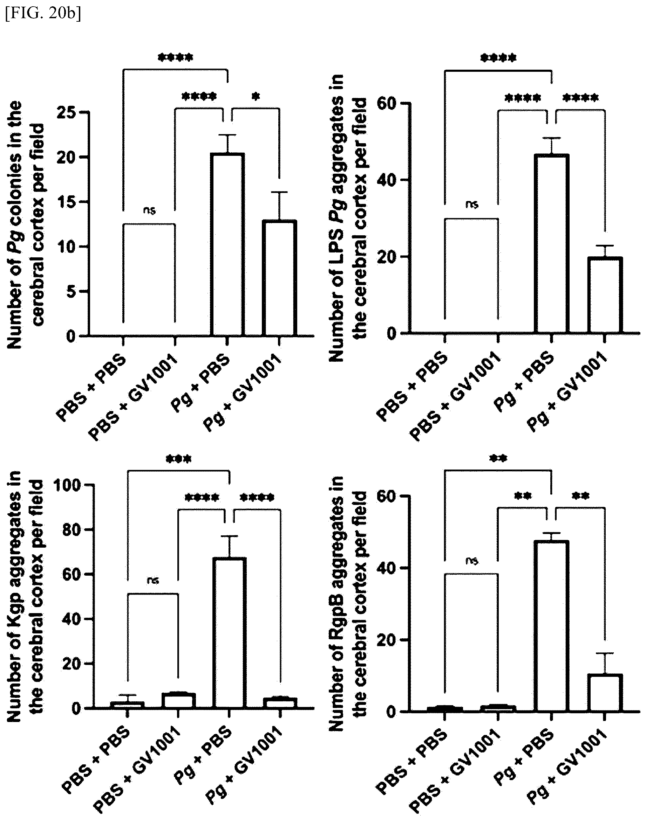

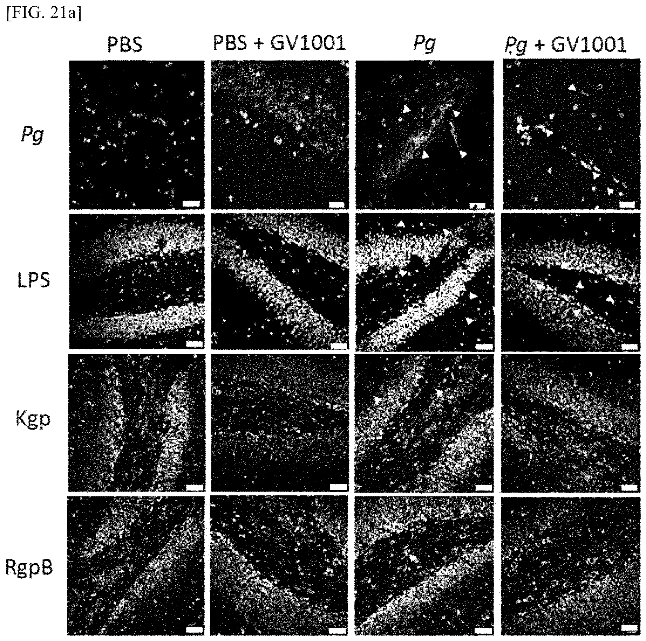

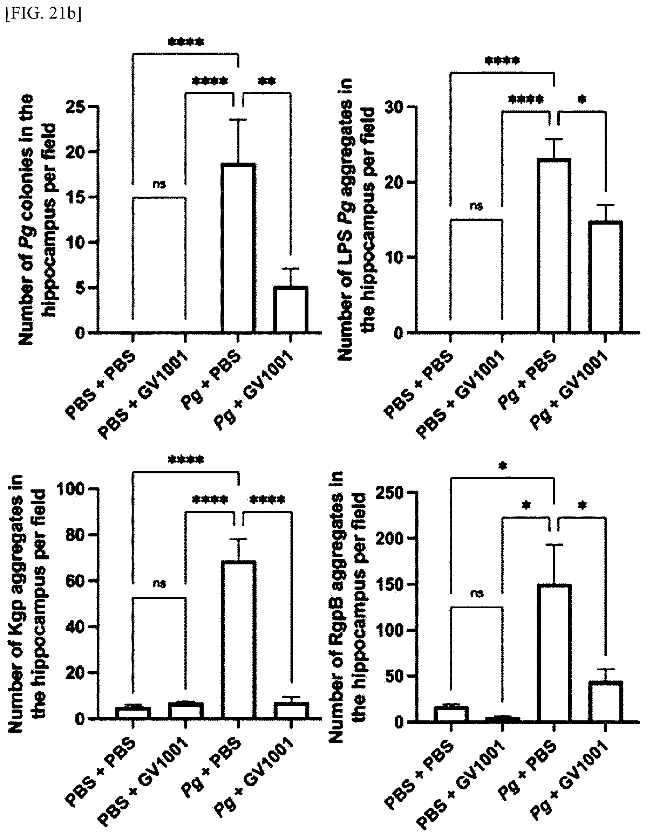

In the cerebral cortex of an animal model of periodontitis, the number of *Porphyromonas gingivalis* colonies and changes in aggregates of *Porphyromonas gingivalis* LPS and gingipain (Kgp, RgpB) were confirmed (FIG. 20). Further, in the hippocampus, the number of *Porphyromonas gingivalis* colonies and changes in aggregates of *Porphyromonas gingivalis* LPS and gingipain (Kgp, RgpB) were also confirmed (FIG. 21).
- Control group: In the periodontitis-induced model (control group), the number of *Porphyromonas gingivalis* colonies, *Porphyromonas gingivalis* LPS, and gingipain in the cerebral cortex and hippocampus were increased.
- GV1001 administration group: It was confirmed that the number of *Porphyromonas gingivalis* colonies, *Porphyromonas gingivalis* LPS, and gingipain in the cerebral cortex and hippocampus, was significantly reduced.

### 3) Aβ₄₂ and p-Tau

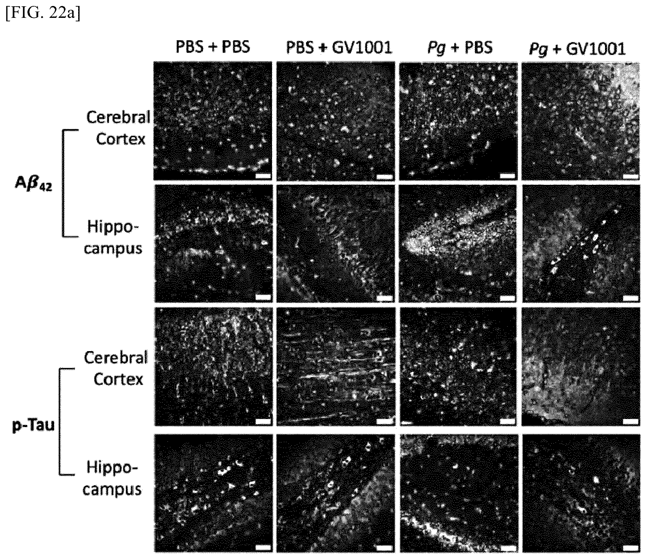

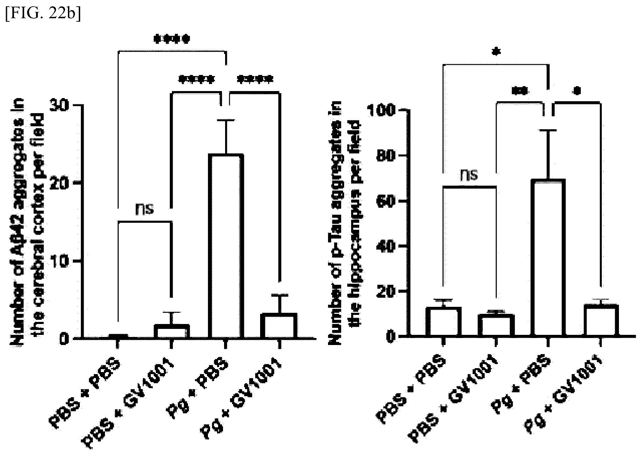

Accumulation of Aβ ₄₂ and p-Tau as biomarkers of Alzheimer's disease was confirmed in the brain of an animal model induced with periodontitis (FIG. 22).
- Control group: In the periodontitis-induced model (control group), accumulation of Aβ₄₂ and p-Tau in the cerebral cortex and hippocampus was increased.
- GV1001 administration group: It was confirmed that the accumulation of Aβ₄₂ and p-Tau in the cerebral cortex and hippocampus was significantly reduced.

### 2.4 Clarification of the mechanism for the inhibition effect of GV1001 on Porphyromonas gingivalis expression

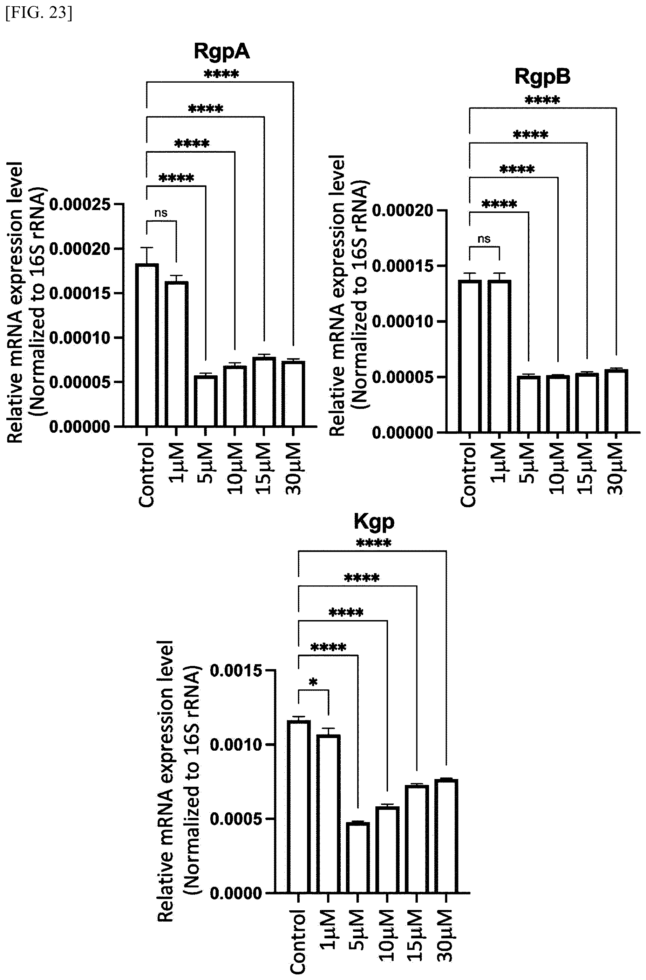

To understand the fundamental mechanism of *Porphyromonas gingivalis* colony formation, and the reduction of *Porphyromonas gingivalis* LPS and gingipain by GV1001 in gingival tissues, arterial walls and brain, the effect of GV1001 on the expression of gingipain, which is an enzyme important for the growth and tissue invasion of *Porphyromonas gingivalis,* was investigated. As a result, GV1001 markedly inhibited the expression of gingipain *in vitro.* Very low doses (5-30 µM) of GV1001 reduced the expression of RgpA and RgpB by approximately 5 times and the expression of Kgp by approximately 3 times, compared with the control group (*Porphyromonas gingivalis* 16S rRNA) (FIG. 23).

### 3. Conclusion

A periodontitis model was established by significantly increasing alveolar bone loss in the periodontitis-induced model treated with *Porphyromonas gingivalis.* It was verified that GV1001 administration can prevent and treat periodontitis by reducing inflammatory cytokines and suppressing osteoclastogenesis.

Further, it was confirmed that, in regard to atherosclerosis induced by lipid deposition in arterial blood vessels was increased by periodontitis, GV1001 decreased inflammatory cytokines in the blood, lipid deposition in the arterial wall and CD47 expression in the arterial root, thereby suppressing the progression of atherosclerosis.

Further, it was confirmed that GV1001 administration reduced inflammatory cytokines in brain tissue and Alzheimer's-related biomarkers (Aβ₄₂ and p-Tau) in Alzheimer's disease induced by periodontitis.

*In vitro* tests confirmed that GV1001 inhibited the expression of gingipain, a protease used by *Porphyromonas gingivalis* for tissue penetration. Through this, it was demonstrated that GV1001 reduced *Porphyromonas gingivalis* colony formation, *Porphyromonas gingivalis* LPS and gingipain in gingival tissue, arterial walls, and brain.

## Claims

1. A pharmaceutical composition for use in preventing or treating periodontal disease, or atherosclerosis or Alzheimer's disease caused by periodontal disease, comprising a peptide having an amino acid sequence of SEQ ID NO: 1.

2. The pharmaceutical composition according to claim 1, wherein the periodontal disease is *Porphyromonas gingivalis-*induced periodontitis.

3. The pharmaceutical composition according to claim 1, wherein the pharmaceutical composition prevents or treats the periodontal disease by suppressing osteoclastogenesis.

4. The pharmaceutical composition according to claim 1, wherein the pharmaceutical composition prevents or treats the periodontal disease by suppressing the expression of gingipain.

5. A kit for use in preventing or treating periodontal disease or atherosclerosis or Alzheimer's disease caused by periodontal disease, comprising:
the pharmaceutical compositions according to any one of claims 1 to 4; and
instructions describing a method for preventing or treating the periodontal disease or the atherosclerosis or Alzheimer's disease caused by periodontal disease.

6. The kit according to claim 5, wherein the method for preventing and treating comprises administering the pharmaceutical composition to a subject who has developed, or is at risk of developing, the periodontal disease, or the atherosclerosis or Alzheimer's disease caused by periodontal disease.

7. A health functional food for use in preventing or improving a periodontal disease, or atherosclerosis or Alzheimer's disease caused by periodontal disease, comprising a peptide having the amino acid sequence of SEQ ID NO: 1.

8. The food according to claim 7, wherein the periodontal disease is *Porphyromonas gingivalis-*induced periodontitis.

9. The food according to claim 7, wherein the health functional food is a health functional food that prevents or improves periodontal disease by suppressing osteoclastogenesis.

10. The food according to claim 7, wherein the health functional food is a health functional food that prevents or improves periodontal disease by suppressing the expression of gingipain.
